# EUROPÄISCHE PATENTANMELDUNG

(11) **EP 4 272 792 A1**
(43) Veröffentlichungstag der Anmeldung: **08.11.2023**
(21) Anmeldenummer: 22171524.6
(22) Anmeldetag: 04.05.2022
(51) Int. Cl.: A61M 16/04, A61B 1/005, A61B 1/018

(54) **MEDIZINISCHES INSTRUMENT FÜR DIE INTUBATION**

(71) Anmelder: Albert-Ludwigs-Universität Freiburg, 79098 Freiburg (DE)
(72) Erfinder: Schick, Priv.-Doz. Dr. med. Martin, 79098 Freiburg (DE); Petzold, Dr. med. Ralf, 79098 Freiburg (DE)
(74) Vertreter: Rösler, Uwe

(57) **Zusammenfassung**

Beschrieben wird ein medizinisches Instrument zum Intubieren eines Patienten mit einem einen Hohlkanal einschließenden Endotrachealtubus, kurz ETT, sowie einem längs des Hohlkanals angeordneten, wenigstens in einem axialen Teilbereich plastisch verformbaren Führungsstab, mit einem distalen und proximalen Stabende, an dessen distalen Stabende ein bidirektional verformbares Führungselement angebracht ist, mit einem distalen und einem proximalen Elementende, dessen proximales Elementende am distalen Stabende gefügt ist, und das mit einem Steuermittel verbunden ist.

Die Erfindung zeichnet sich dadurch aus, dass am proximalen Stabende des Führungsstabes eine Bedieneinheit angebracht ist, mit einem mit dem Steuermittel verbundenen Aktuator sowie mit einem lösbar fest mit dem ETT in Wirkverbindung stehenden Schub-Zug-Mechanismus, durch den der ETT längs des Führungsstabes bewegbar ist.

## Beschreibung

### Technisches Gebiet

Die Erfindung bezieht sich auf ein medizinisches Instrument zum Intubieren eines Patienten mit einem einen Hohlkanal einschließenden Endotrachealtubus, kurz ETT, sowie einem längs des Hohlkanals angeordneten, wenigstens in einem axialen Teilbereich plastisch verformbaren Führungsstab, mit einem distalen und proximalen Stabende, an dessen distalen Stabende ein bidirektional verformbares Führungselement angebracht ist, mit einem distalen und einem proximalen Elementende, dessen proximales Elementende am distalen Stabende gefügt ist, und das mit einem Steuermittel in Wirkverbindung steht.

Für die Sicherung des Atemweges für eine Allgemeinanästhesie ist und bleibt die Intubationsnarkose, kurz ITN, ein Standardprocedere, für das verschiedene Hilfsmittel benötigt werden, um einen Beatmungsschlauch bzw. Endotrachealtubus, kurz ETT, in die Luftröhre des Patienten zu platzieren. Hierbei muss der ETT durch den offenen Mund, Rachen und nachfolgend zwischen den Stimmbändern hindurch in die Luftröhre (Trachea) vorgeschoben werden. Bei diesem Procedere ist die Sicht auf die Stimmbänder, welche in aller Regel unter Zuhilfenahme eines Laryngoskops hergestellt wird, elementar. Während dieser Phase kann der Patient nicht beatmet werden, so dass ein zügiges und reibungsloses Procedere für den Patienten sehr wichtig, wenn nicht gar überlebenswichtig ist. Erst über den korrekt platzierten ETT kann der Patient sicher beatmet werden.

Ist die Intubation auf Anhieb nicht oder nur erschwert möglich, so spricht man von einer schwierigen Atemwegssicherung bzw. schwierigen Intubation. Bei Erwachsenen tritt dieser Fall in 3-10% aller durchgeführten Intubationsfällen auf, siehe Ruetzler K, Smereka J, Abelairas-Gomez C, Frass M, Dabrowski M, Bialka S, et al., Comparison of the new flexible tip bougie catheter and standard bougie stylet for tracheal intubation by anesthesiologists in different difficult airway scenarios: a randomized crossover trial. BMC Anesthesiol. 2020;20(1):90.

Allein in Deutschland werden pro Jahr ca. 24 Mio. Operationen durchgeführt, von denen etwa 70% durch eine Allgemeinanästhesie unterstützt und begleitet werden, so dass ca. 0,5 bis 1,6 Millionen Patienten pro Jahr in Deutschland von dieser Problematik betroffen sind.

Zur Erleichterung und Unterstützung der Einführung und Platzierung des ETT im Rachen sowie durch die Stimmbänder hindurch in den oberen Luftröhrenbereich hineinragend, wird ein sogenannter Führungsstab (engl. bougie, stylet) entweder zur Formgebung innerhalb des ETT platziert oder anstelle des ETT in die Luftröhre vorab eingeführt, um unmittelbar nachfolgend den ETT über den bereits eingelegten Führungsstab in die Luftröhre vorzuschieben. Während des gesamten Intubationsprozesses kann der Patient nicht beatmet werden. Zur Sicherstellung der Sauerstoffversorgung des Patienten kann es während des Intubationsprozesses immer wieder nötig werden, den Patienten z.B. mit einer Maske zwischenzubeatmen.

Der Einsatz eines Führungsstabes ist vor allem auch in jenen Fällen besonders vorteilhaft, in denen zwar, zumeist unter Verwendung eines Laryngoskops, eine gute Sicht auf die Stimmbänder möglich ist, jedoch trotz einer geeigneten Patientenkopflage die orale, pharyngeale sowie die tracheale Atemwegsachse des Patienten ungünstig zueinander orientiert sind, so dass der weiche und produktionsbedingt vorgeformte Tubus in nicht geeigneter Weise platzierbar ist.

Der Führungsstab wird in diesen Fällen an die anatomischen Gegebenheiten individuell empirisch vorgebogen, was häufig dazu führt, dass mehrere Intubationsversuche mit im ETT einliegenden, vorgebogenen Führungsstab vorgenommen werden müssen, dessen Biegung nach jedem misslungenen Intubationsversuch angepasst werden muss, bis der richtige Winkel gefunden worden ist und der ETT in die Trachea platziert werden kann. Dieses Procedere kann mitunter mehrere Minuten benötigen.

Eine weitere Anwendung des Führungsstabes kommt bei der sogenannten Rapid Sequence Induction (RSI) zum Tragen. Dieser Prozess beinhaltet eine schnelle Intubation von Patienten nach der Narkoseeinleitung, welche z.B. hochgradig von einer Aspiration gefährdet sind, d.h. Erbrechen von Mageninhalt und anschließendem Eindringen dieser Bestandteile in die Lunge. Um hier eine schnelle und erfolgreiche Intubation zu ermöglichen, wird bei dem ersten Versuch ein Führungsstab standardmäßig in den ETT platziert und anhand der mutmaßlichen Anatomie vorgeformt. Auch hier kann es, wie vorstehend erwähnt, vorkommen, dass der Intubationsvorgang abgebrochen werden und der Führungsstab samt ETT neu vorgebogen werden muss. Hierbei besteht nicht nur die Gefahr einer Sauerstoffminderversorgung durch die fehlende Beatmung, sondern zusätzlich einer potenziell tödlichen Aspiration.

Nach einer erfolgreichen Platzierung des ETT inkl. Führungsstab, üblicherweise kurz vor oder nach der Stimmbandebene, wird der Führungsstab durch eine zweite Person entweder schrittweise zurückgezogen oder vollständig entfernt. Bei diesem Vorgang kann es vorkommen, dass durch diese Manipulation der korrekt platzierte ETT aufgrund der zwischen dem ETT und dem Führungsstab vorherrschenden hohen Reibungskräften verrutscht und dadurch nicht mehr in die Trachea einführbar ist. Den Führungsstab in situ wieder in den ETT einzuführen gelingt in den wenigsten Fällen, so dass der Vorgang abgebrochen und der Prozess von Anfang an neu begonnen werden muss, mit all den damit vorstehend erläuterten Risiken.

Der Führungsstab wird für die Mindestausstattung eines Anästhesiearbeitsplatzes der Deutschen Gesellschaft für Anästhesiologie und Intensivmedizin gefordert (S1 Leitlinie Atemwegsmanagement, siehe www.awmf.org).

### Stand der Technik

Neben der plastischen Verformbarkeit des Führungsstabes, durch die eine Vorbiegung des Führungsstabes manuell vorgenommen werden kann, um dessen Einführung und Platzierung durch den Rachen sowie die Stimmbänder hindurch in den oberen Luftröhrenbereich zu erleichtern, weisen sehr wenige moderne Führungsstäbe darüber hinaus jeweils einen kontrolliert verformbaren, distalen Stabendbereich auf, dessen räumliche Krümmung zum Zwecke einer erleichterten und Patienten schonenden Einführung des Führungsstabes veränderbar ist.

In der Druckschrift EP 3 065 803 B1 ist ein Führungsstab bzw. Bougie offenbart, der einen rohrförmigen, plastisch verformbaren Hauptschaft aufweist, an dessen distalen Ende eine bewegliche Spitze angebracht ist, die mittels eines innerhalb des Hauptschaftes längsbeweglich gelagertes Steuerglied in einer Verformungsebene bidirektional verformbar ist. Hierzu sieht der rohrförmige Hauptschaft eine sich in Längsrichtung erstreckende Ausnehmung vor, durch die mit dem Steuerglied verbundene Vorsprünge ragen, die für den Bediener manuell zugänglich sind.

Die Druckschrift EP 3 528 878 B1 beschreibt einen gelenkigen Mandrin zur Anwendung mit einem endotrachealen Tubus, wobei der Mandrin über einen flexiblen, distalen Spitzenabschnitt verfügt, der in einer ersten Richtung vorgespannt ist und über einen Steuerdraht, der den distalen Spitzenabschnitt mit einem proximalen Mandrinabschnitt verbindet, durch entsprechende Zugwirkung in eine zur ersten Richtung entgegengesetzten zweiten Richtung verformbar ist.

Ein zum vorstehenden Mandrin sehr ähnlicher Führungsstab ist der Druckschrift WO 2007/138569 A2 zu entnehmen, der einen starren sowie einen sich daran anschließenden flexiblen Stababschnitt aufweist, an dessen distalen Ende ein, um eine Achse schwenkbar gelagertes Schwenkelement angebracht ist, zu dessen einseitiger Auslenkung ein Zugkräfte übertragendes Drahtseil vorgesehen ist, das einerseits mit dem Schwenkelement und andererseits mit einem proximalseitig am Führungsstab angebrachten Hebelmechanismus verbunden ist. Zur Intubation wird der Führungsstab gemeinsam mit dem diesem umgebenden Endotracheal-Tubus oral in den Patienten eingeführt.

Die Druckschrift WO 2017/079434 A1 offenbart einen Führungsstab mit einer elastisch verformbaren distalen Führungsstabspitze, an der wenigstens ein, vorzugsweise mehrere Zugkräfte übertragende Drähte angebracht sind, die mit einem als Joy-Stick-Körper am proximalen Führungsstabende angebrachten Griffkörper verbunden sind. Durch Verdrehen oder Neigen des Joy-Stick-Körpers erfahren die Zugdrähte entsprechende Längsverschiebungen, deren Auslenkungen zur Verformung der Spitze des Führungsstabes dienen.

Bei derzeit bekannten Führungsstäben, die in den Endotracheal-Tubus platziert werden, muss der Führungsstab entweder von einer zweiten Person im Intubationsvorgang entfernt werden, oder der ETT muss von einer zweiten Person vorgeschoben werden, oder das Laryngoskop muss entfernt werden, um den ETT daraufhin blind vorschieben zu können. Dies resultiert aus der Tatsache, dass die Person, die den Patienten intubiert, in der linken Hand das Laryngoskop und in der rechten Hand den einzuführenden ETT hält. Dadurch sind beide Hände okkupiert und es fehlt in jedem Falle eine dritte Hand.

### Darstellung der Erfindung

Der Erfindung liegt die Aufgabe zugrunde, ein medizinisches Instrument zum Intubieren eines Patienten mit einem einen Hohlkanal einschließenden ETT sowie einen längs des Hohlkanals angeordneten, wenigstens in einem axialen Teilbereich plastisch verformbaren Führungsstab mit einem distalen und einem proximalen Stabende, an dessen distalen Stabende ein bidirektional verformbares Führungselement angebracht ist, mit einem distalen und mit einem proximalen Elementende, dessen proximales Elementende am distalen Stabende gefügt ist und das mit einem Steuermittel in Wirkverbindung steht, derart weiterzubilden, so dass die Handhabung des medizinischen Instruments zur Durchführung der gesamten Intubationsprozedur, d.h. Einführen und Platzieren des ETTs mittels des Führungsstabes sowie Separieren des Führungsstabes vom ETT und Entnehmen desselben, durch die intubierende Person einhändig durchführbar sein soll, so dass zu keinem Zeitpunkt eine dritte Hand benötig wird und infolgedessen auch keine Notwendigkeit für eine zweite, den Intubationsvorgang unterstützende Person besteht.

Zur Lösung der der Erfindung zugrunde liegenden Aufgabe ist ein medizinisches Instrument zum Intubieren eines Patienten mit den Merkmalen des Oberbegriffes des Anspruches 1 dadurch ausgebildet, dass am proximalen Stabende des Führungsstabes eine Bedieneinheit angebracht ist, mit einem mit dem Steuermittel verbundenen Aktuator sowie mit einem lösbar fest mit dem ETT in Wirkverbindung stehenden Schub-Zug-Mechanismus, durch den der ETT längs des Führungsstabes bewegbar ist.

Mit dem lösungsgemäßen medizinischen Instrument ist erstmals für eine intubierende Person die Möglichkeit geschaffen, nach entsprechend manueller, auf empirischer Erfahrungen basierender Vorformung des Führungsstabes diesen zusammen mit dem ETT, d.h. der Führungsstab ist innerhalb des ETT angeordnet, mit nur einer Hand innerhalb des sich an den Rachen anschließenden Atemwegbereichs durch die Stimmbänder hindurch schonend und zielgerichtet zu navigieren und in einer entsprechenden korrekten Position zu platzieren, wobei der ETT auf diese Art aktiv über den Führungsstab verformt werden kann. So ist das am distalen Stabende bidirektional verformbare Führungselement mit Hilfe des an der Bedieneinheit angebrachten Aktors, vorzugsweise mit Hilfe eines einzigen Fingers, beispielsweise des Daumens, kontrolliert bidirektional verformbar bzw. krümmbar, so dass das distale Elementende je nach Auslenkung entweder in eine Richtung oder in die entsprechende Gegenrichtung auslenkbar ist, jeweils in Abhängigkeit der Patienten-spezifischen Atemwegsgeometrie.

Zudem bietet das lösungsgemäß ausgebildete medizinische Instrument für die intubierende Person die Möglichkeit, den ETT über den Führungsstab in die Trachea zu platzieren und diesen anschließend vom Führungsstab zu separieren, ohne dass ein manuelles Umgreifen oder die Hilfestellung einer weiteren Person erforderlich wird. Gleichsam der Aktuatorbedienung ist zum Zwecke eines distalwärtigen Vorschubes des ETT über den Führungsstab in die Luftröhre ein an der Bedieneinheit angebrachter Schub-Zug-Mechanismus vorgesehen, der gleichsam der vorstehend erläuterten Aktuatorbedienung auch mit Hilfe nur eines Fingers, vorzugsweise mit Hilfe des Zeigefingers, bedienbar ist.

Die vorteilhafte Einhandbedienung des lösungsgemäßen, medizinischen Instrumentes ist besonders geeignet für medizinische Notfalleinsätze, bei denen eine zweite Person fehlt oder anderweitig gebunden ist. Durch eine bevorzugte ergonomische Ausbildung der Bedieneinheit mit den daran angebrachten manuell betätigbaren Aktuator und Schub-Zug-Mechanismus sind instrumentelle Voraussetzungen für ein schnelles und schonendes Intubieren geschaffen, wodurch ein Unterbrechen bzw. Abbrechen von Intubationsvorgängen aufgrund von Aspiration und/oder Sauerstoff-Minderversorgung ausgeschlossen oder zumindest signifikant reduziert werden können.

Das "einhändige" Bedienkonzept des lösungsgemäßen medizinischen Instruments zum Intubieren eröffnet zudem die Möglichkeit eines Robotereinsatzes, der insbesondere in Pandemie-Zeiten, beispielsweise bedingt durch Corona-Viren, wie (SARS-CoV-2, SARS, MERS) oder Influenza, Ebola, von großem Interesse ist, um die intubierenden Personen zu schützen. Hierzu gilt es die Bedieneinheit in geeigneter Weise in Form eines mechanischen oder mechatronischen Verbindungsflansches zum Zwecke der Adaption an und Betätigung durch eine Robotereinheit auszubilden.

In einer bevorzugten Ausführungsform des medizinischen Instrumentes weist das einerseits mit dem Aktuator und andererseits mit dem distalen Elementende des bidirektional verformbaren Führungselementes verbundene Steuermittel wenigstens zwei Zugkräfte übertragende, vorzugsweise seil- oder bandförmig ausgebildete Strangabschnitte auf, die mit dem Aktuator derart in Wirkverbindung stehen, so dass bei Betätigung des Aktuators beide Strangabschnitte entgegengesetzt zueinander kinematisch auslenkbar sind. Hierzu weist der Aktuator vorzugsweise ein um eine Drehachse drehbar gelagertes Drehelement auf, an dem jeweils die Strangendabschnitte beider Strangabschnitte in zur Drehachse gegenüber liegenden Halbebenen mit dem Drehelement in Wirkverbindung stehen. Die distalseitigen Strangendabschnitte der zwei Zugkräfte übertragenden Strangabschnitte sind jeweils am distalen Elementende des vorzugsweise aus biegeelastischem Material gefertigten Führungselementes an zwei zur Längsachse des biegeelastischen Führungselementes gegenüberliegenden Befestigungsstellen angebracht.

Beide Strangabschnitte sind vorzugsweise gleich lang dimensioniert und nehmen in einer Neutralstellung, in der das vorzugsweise länglich biegeelastisch ausgebildete Führungselement eine geradlinige, d.h. krümmungsfreie Form einnimmt, jeweils einen weitgehendgespannten und Zugkräfte-freien Zustand ein. Bei Betätigung des Aktors, d.h. bei Rotation des Drehelementes, wird einer der beiden Strangabschnitte unter Ausbildung einer auf das Führungselement wirkenden Zugkraft proximalwärts um eine Wegstrecke ausgelenkt, längs der der gegenüber liegende Strangabschnitt distalwärts geführt wird. Im Ergebnis krümmt sich das biegeelastische Führungselement in Richtung des Zugkraft-beaufschlagten Strangabschnittes. In der gleichen Weise erfolgt eine Krümmung des Führungselementes in die exakt entgegengesetzt orientierte Richtung, bei Betätigung des Aktors und Drehung des Drehelementes in entgegengesetzter Richtung um die Drehachse.

In einer weiteren Ausführungsform sind die mit dem Drehelement in Wirkverbindung stehenden Strangendabschnitte beider Strangabschnitte einstückig bzw. monolithisch miteinander verbunden. Das Drehelement ist in diesem Fall in Art einer Drehscheibe mit einem nutförmig ausgebildeten Umfangsrand ausgebildet, längs dessen Umfangsrandhälfte das seil- oder bandförmig ausgebildete Steuermittel enganliegend umläuft.

Alternativ zur Ausbildung des Steuermittels in Art einer mechanischen Bowdenzug-Lösung, die mit dem biegeelastisch ausgebildeten Führungselement zusammenwirkt, wie vorstehend beschrieben, bietet es sich ebenso an ein geeignet konfektioniertes, biegeelastisches Führungselement mittels elektrischer, pneumatischer oder hydraulischer Steuersignale kontrolliert zu krümmen bzw. zu verformen. Hierzu weist das bidirektional verformbare Führungselement eine Baueinheit auf, die durch elektrische, pneumatische oder hydraulische Steuersignale eine Formänderung des Führungselementes zu initiieren vermag.

In einer Ausführungsvariante weist die Baueinheit wenigstens eine der nachfolgenden, mittels elektrischer Steuersignale aktivierbare elektrische bzw. mechatronische Komponenten auf: elektromotorische Getriebeeinheit, Aktor-Sensor-Einheit, piezoelektrischer Werkstoff, magnetostriktiver Werkstoff, Formgedächtnislegierung, etc.. Beispielsweise im Falle der Verwendung von piezioelektrischen Materials, das als integraler Bestandteil an, längs und/oder innerhalb des Führungselementes vorgesehen ist, führen elektrisch initiierte Materialkontraktionen zu Formänderungen des Führungselementes. Die elektrischen Steuersignale sind durch einen elektrischen Schaltkreis, wenigstens enthaltend eine elektr. Energiequelle sowie eine Schaltereinheit, an das piezoelektrische Material applizierbar. Vorzugsweise sind die elektr. Energiequelle sowie die als Aktuator dienende Schaltereinheit in der Bedieneinheit untergebracht. Längs des Führungsstabes verlaufen lediglich zwei elektrische Leitungswege, um die jeweils im Führungselement zum Zwecke dessen Formänderung integrierte elektrische Komponente anzusteuern.

In gleicher Weise sind in alternativen Ausführungsbeispielen innerhalb des Führungselementes integrierte pneumatisch oder hydraulisch aktivierbare Komponenten, bspw. in Form wenigstens eines Bläh- oder Füllkörpers bzw. -volumens, mittels längs des Führungsstabes verlaufenden Fluidleitungen ansteuerbar. In diesen Fällen enthält der hierzu geeignet konfektionierte pneumatische oder hydraulische Schaltkreislauf bzw. Fluidkreislauf über eine Pumpeneinheit sowie ein als Aktuator dienendes Steuerventil, die in geeigneter Weise Teil der Bedieneinheit sind.

Zum Zwecke einer möglichst komfortablen und ermüdungsfreien Handhabung des medizinischen Instrumentes ist in einer bevorzugten Ausführungsform die als Handgriff ausgebildete Bedieneinheit in Art eines ergonomisch geformten Umgreifungskörpers ausgebildet, der einseitig vom Daumen und gegenüber liegend von der übrigen Hand umschlossen werden kann. Zur Betätigung des Aktuators sieht dieser wenigstens ein erstes Bedienelement vor, das derart am Umgreifungskörper ergonomisch angeordnet und ausgebildet ist, dass das erste Bedienelement mittels des Daumens einer den Umgreifungskörper umschließenden Hand betätigbar ist. Hierzu ist das erste Bedienelement vorzugsweise in Art eines Hebels oder Stellrades ausgebildet, das vom Daumen entweder in die eine oder die entgegengesetzte Richtung schwenkbar bzw. verstellbar ist.

Darüber hinaus ist am ergonomisch ausgebildeten Handgriff ein zweites Bedienelement derart ergonomisch angeordnet und ausgebildet, dass das zweite Bedienelement mittels des Zeige- bzw. Mittelfingers der den Handgriff umschließenden Hand betätigbar ist. Das zweite Bedienelement dient zur Betätigung des Schub-Zug-Mechanismus, durch den der ETT längs des Führungsstabes bewegbar ist. Das zweite Bedienelement ist hierzu vorzugsweise in Art eines am Handgriff angebrachten Hebels ausgebildet, der vermittels des Zeige- bzw. Mittelfingers sowohl in Richtung des Handgriffes gezogen werden kann, als auch in entgegengesetzter Richtung vom Handgriff weggedrückt werden kann. Vorzugsweise ist das zweite Bedienelement ringförmig ausgebildet, durch das der Zeige- bzw. Mittelfinger hindurch gesteckt werden kann.

Das in der vorstehenden Weise ausgebildete zweite Bedienelement ist über eine im Handgriff angeordnete Getriebeeinheit mit einer Schub-Zug-Stange kinematisch verbunden, die lösbar fest mit dem proximalen Bereich des ETT gefügt ist. Die Getriebeeinheit vermag bei proximalwärtiger Auslenkung des zweiten Bedienelementes in Richtung des Handgriffes die Schub-Zug-Stange sowie den mit dieser lösbar fest gefügten ETT distalwärts und bei entgegengesetzter Auslenkung des zweiten Bedienelementes die Schub-Zug-Stange sowie den mit dieser lösbar fest gefügten ETT proximalwärts auszulenken bzw. zu schieben. Vorzugsweise sieht die Getriebeeinheit ein um eine Drehachse drehbar gelagertes Zahnrad mit Außenverzahnung vor, in die sowohl eine mit dem zweiten Bedienelement verbundene Zahnstange als auch die in Art einer Zahnstange ausgebildete Schub-Zug-Stange eingreifen. Eine bevorzugte Ausführungsform zur konstruktiven Ausgestaltung der Getriebeeinheit ist nachstehend unter Bezugnahme auf die Figuren detailliert illustriert und beschrieben.

Der zumindest bereichsweise plastisch verformbare Führungsstab weist eine Länge auf, die wenigstens der Länge des ETT's entspricht. Aufgrund der Materialinhärenten Flexibilität des ETT's um dessen Längsachse ist es vorteilhaft, jedoch nicht erforderlich, dass das biegeelastische am proximalen Ende des Führungsstabes angebrachte Führungselement distalseitig über den ETT ragt.

In einer weiteren Ausführungsform ist längs des Lumens durch den Führungsstab, durch das die Strangabschnitte des Steuermittels geführt sind, oder eines weiteren Lumens durch den Führungsstab ein Beobachtungsmittels geführt, das beispielsweise in Form eines Beleuchtungs-Katheters, eines Kamera-Katheters oder eines LIDAR-Sensor unterstützten Katheters ausgebildet sein kann. Auf diese Weise ist es möglich, den Intubationsvorgang visuell überwacht durchzuführen und die kontrolliert vorgebbare Krümmung des Führungselementes in situ individuell an die räumlichen Atemwegs-Gegebenheiten anzupassen, um den ETT schonend und schnellstmöglich zu platzieren.

### Kurze Beschreibung der Erfindung

Die Erfindung wird nachstehend ohne Beschränkung des allgemeinen Erfindungsgedankens anhand von Ausführungsbeispielen unter Bezugnahme auf die Zeichnung exemplarisch beschrieben. Es zeigen:
Fig. 1 Ausführungsbeispiel eines erfindungsgemäßen medizinischen Instrumentes zum Intubieren eines Patienten,
Fig. 2 Führungsstab mit bidirektional verformbaren Führungselement,
Fig. 3 Detaildarstellung des verformbaren Führungselementes sowie
Fig. 4 Führungsstab mit bidirektional verformbaren Führungselement mit elektrisch, pneumatisch oder hydraulisch aktivierbarer Baueinheit.

### Wege zur Ausführung der Erfindung, gewerbliche Verwendbarkeit

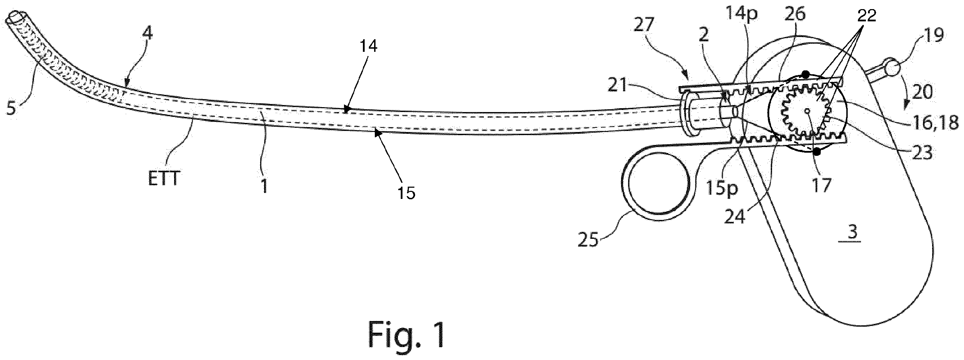

In Figur 1 ist eine schematisierte Gesamtdarstellung des medizinischen Instrumentes zum Intubieren eines Patienten mit ETT illustriert, das einen Führungsstab 1 aufweist, dessen proximales Stabende 2 mit einer als Handgriff ausgebildeten Bedieneinheit 3 verbunden ist und an dessen distalen Stabende 4 ein bidirektional verformbares Führungselement 5 angeordnet ist.

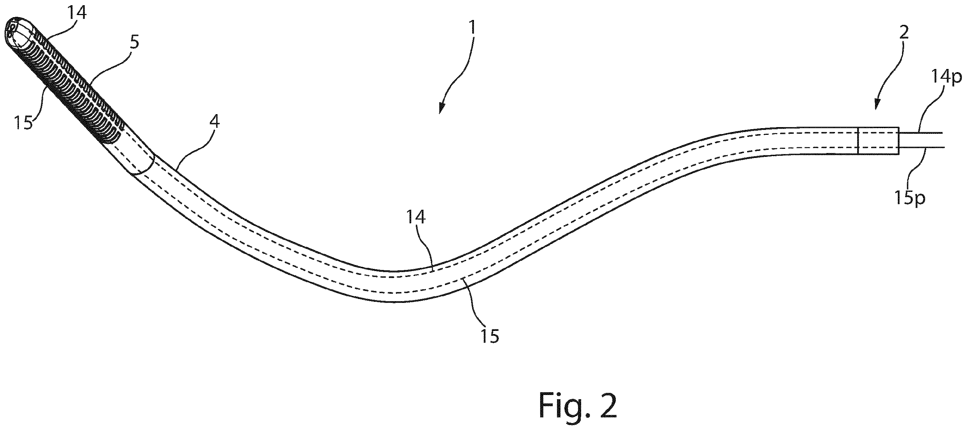

In Figur 2 ist der Führungsstab 1 sowie das an dessen distalen Stabende 4 angeordnete bidirektional verformbare Führungselement 5 in Alleindarstellung illustriert.

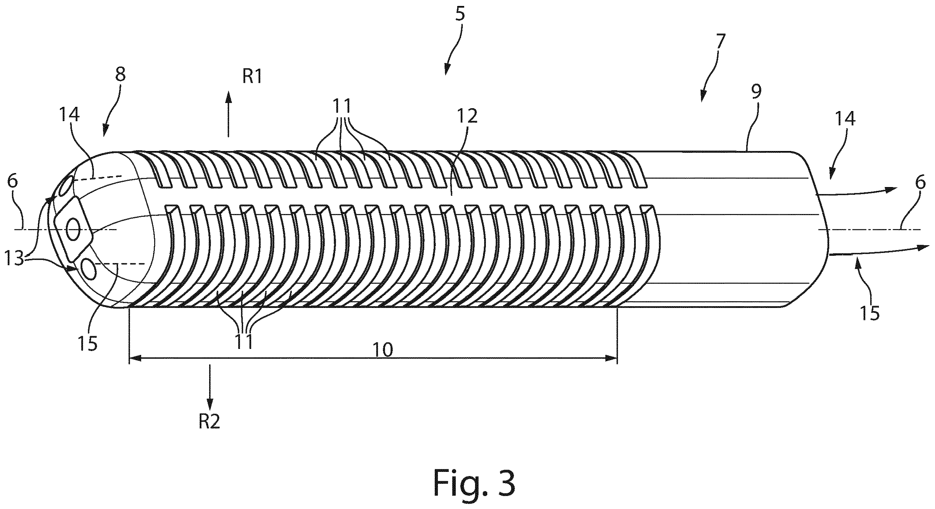

Figur 3 zeigt eine bevorzugte Ausführungsform eines bidirektional verformbaren Führungselementes 5 in Detailansicht. Die weiteren Ausführungen nehmen gemeinsamen Bezug auf die Figuren 1 bis 3.

Der als Hohlkanal ausgebildete Führungsstab 1 ist vorzugsweise aus einem plastisch verformbaren Material gefertigt, so dass eine intubierende Person den Führungsstab 1 zu Zwecken einer möglichst patientenschonenden oralen Einführung in den Rachenbereich sowie den daran anschließenden Atemweg manuell vorzukrümmen vermag, wie dies in Figur 2 dargestellt ist.

Das am distalen Stabende 4 gefügte bidirektional verformbare Führungselement 5 besteht gemäß dem in Figur 3 im Detail dargestellten Ausführungsbeispiel aus einem monolithisch gefertigten hülsenartig ausgebildeten Hohlkörper, dem eine Längsachse 6, ein proximales Elementende 7 sowie ein distales Elementende 8 zugeordnet ist. Das proximale Elementende 7 ist einseitig fest mit dem distalen Stabende 4 des Führungsstabes 1 gefügt. Das distale Elementende 8 ist kuppenartig ausgebildet und verfügt über eine weitgehend geschlossene Oberfläche, um ein möglichst läsionsfreies und gleitendes Eindringen des Führungsstabes 1 in den Atemwegsbereich bzw. zum einfach Einführen des Führungsstabes in den ETT zu gewährleisten.

Das in Figur 3 illustrierte Führungselement 5 stellt einen zur Längsachse 6 symmetrischen Hohlkörper dar. Im Bereich des proximalen Elementendes 7 weist das Führungselement 5 zu Zwecken einer möglichst formstabilen Fügung an den Führungsstab 1 eine unterbrechungsfreie Hülsenwand 9 auf, an die sich distalwärts ein bidirektional verformbarer Abschnitt 10 anschließt. In diesem Abschnitt 10 ist die Hülsenwand strukturiert und weist jeweils orthogonal zur Längsachse 6 durch Abstände axial voneinander getrennte rippenbogenartig ausgebildete Wandabschnitte 11 auf, die jeweils beidseitig mit einem Hülsenwandsteg 12, der sich jeweils von der proximalseitig unterbrechungsfreien Hülsenwand 9 bis zum distalen Elementende 8 erstreckt, verbunden sind. Der Ordnung halber sei darauf hingewiesen, dass gegenüber dem in Figur 3 sichtbaren Hülsenwandsteg 12 ein entsprechender Hülsenwandsteg an der nicht in Figur 3 sichtbaren Rückseite des Führungselementes 5 angeordnet ist.

Der in der vorstehenden Weise strukturierte und in Figur 3 dargestellte, bidirektional verformbare Abschnitt 10 ermöglicht eine Krümmung des Führungselementes 5 in die beiden, durch Pfeile dargestellten, entgegengesetzt zueinander orientierten Richtungen R1, R2, die jeweils quer zur Längsachse 6 orientiert sind.

Zur kontrollierten Auslenkung des bidirektional verformbaren Führungselementes 5 jeweils längs eines der beiden Krümmungsrichtungen R1, R2 ist am distalen Elementende 8 eine Befestigungsstruktur 13 zur Fixierung der jeweils distalen Elementenden, der zwei Zugkräfte übertragenden Strangabschnitte 14, 15 angebracht. Die Strangabschnitte 14, 15 sind seil- oder bandförmig ausgebildet und verlaufen im Inneren des als Hohlkörper ausgebildeten Führungselementes 5 proximalwärts durch den sich proximalwärts zum Führungselement 5 anschließenden, als Hohlkanal ausgebildeten Führungsstab 1, siehe auch Figur 2.

Die proximalen Strangendabschnitte 14p, 15p der Strangabschnitte 14, 15, siehe Figur 1, ragen proximalseitig aus dem Führungsstab 1 und sind mit einem in der als Handgriff ausgebildeten Bedieneinheit 3 integrierten Aktuator 16 verbunden. Der in Figur 1 illustrierte Aktuator 16 weist ein um eine Drehachse 17 drehbar gelagertes Drehelement 18 auf, an dem ein Bedienhebel 19 angelenkt ist, der mittels des Daumens einer den Handgriff 3 umschließenden Hand bedienbar ist. Bei Schwenken des Bedienhebels 19 in die in Figur 1 dargestellte Schwenkrichtung 20 wird der obere Strangabschnitt 14 proximalwärts gezogen, wohingegen der untere Strangabschnitt 15 distalwärts geführt wird. In diesem Fall wirkt längs des oberen Strangabschnittes 14 eine Zugkraft, die das distalseitig am Führungsstab 1 angebrachte Führungselement 5 in der in Figur 1 dargestellten Weise zu krümmen vermag. Bei Auslenkung des Bedienhebels 19 in die zur Schwenkrichtung 20 entgegengesetzte Schwenkrichtung erfolgt eine entsprechende Krümmungsumkehr des Führungselementes 5.

In Figur 1 ist über den Führungsstab 1 ein Endotracheal-Tubus, kurz ETT, geschoben, an dessen proximalen Ende ein ringförmiger Konnektor 21 angebracht ist, der Bestandteil des ETTs ist. Der Konnektor 21 steht in Wirkverbindung mit einem im Handgriff 3 ebenfalls integrierten Schub-Zug-Mechanismus 22, der durch manuelle Betätigung den ETT längs des Führungsstabes 1 auszulenken vermag. Der Schub-Zug-Mechanismus 22 weist ein um die Drehachse 17 drehbar gelagertes Zahnrad 23 mit Außenverzahnung auf, in die einerseits eine Zahnstange 24 eingreift, die einseitig als ringförmiges, zweites Bedienelement 25 ausgebildet ist, in die der Zeigefinger einer den Handgriff 3 umfassenden Hand eingreifen kann. Zum anderen greift eine weitere Zahnstange 26 gegenüberliegend zur Zahnstange 24 in die Außenverzahnung des Zahnrades 23 ein, die endseitig über einen Rastmechanismus 27 mit dem Kragen 21 des ETTs in lösbar fester Wirkverbindung steht.

Durch proximalwärtige Auslenkung der Zahnstange 24 wird in gleichem Maße die Zahnstange 26 und der mit dieser verbundene ETT distalwärts geschoben. Gleichsam ist es möglich, durch distalwärtige Auslenkung der Zahnstange 24 vermittels des zweiten Bedienelementes 25 den ETT proximalwärts längs des Führungsstabs 1 zu bewegen.

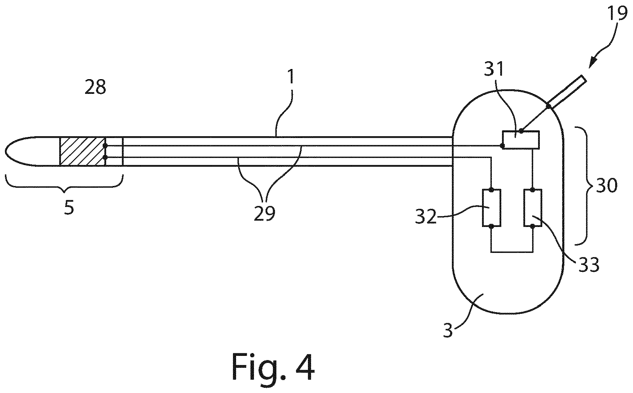

Somit ist es möglich, mit nur einer Hand das medizinische Instrument mit vorgeformtem Führungsstab 1 in den Atemweg einer Person einzuführen und durch geeignete Betätigung des ersten Bedienhebels 19, vorzugsweise mit dem Daumen, das bidirektional verformbare Führungselement 5 zu Zwecken einer patientenschonenden Instrumenteneinführung und erleichterten Navigation individuell bidirektional zu krümmen. Nach entsprechender Platzierung kann sodann der ETT vermittels des zweiten Bedienelementes 25, das in geeigneter Weise mit dem Zeigefinger betätigbar ist, distalwärts in die Trachea vorgeschoben werden. Nach erfolgter Platzierung und Fixierung des ETTs, bspw. in an sich bekannter Weise mit einem am ETT angebrachten inflatierbaren Ballon (sog. Cuff, nicht dargestellt), wird der Rastmechanismus 27 von dem fest mit dem ETT verbundenen Kragen 21 gelöst, und der Führungsstab 1 proximalwärts aus dem ETT gezogen. Figur 4 illustriert einen Führungsstab 1 mit einer proximalseitig angebrachten, als Handgriff ausgebildeten Bedieneinheit 3. Distalseitig am Führungsstab 1 ist ein elastisch biegsames Führungselement 5 vorgesehen, das über eine Baueinheit 28 als integrale Komponente verfügt, die durch elektrische, pneumatische oder hydraulische Steuersignale eine Formänderung des Führungselementes 5 zu initiieren vermag. Zur weiteren Erläuterung sei angenommen, dass die Baueinheit 28 elektrisch betreibbar und aktivierbar ist und in Form einer der nachfolgenden Komponenten ausgebildet ist: elektromotorische Getriebeeinheit, Aktor-Sensor-Einheit, piezoelektrischer Werkstoff, magnetostriktiver Werkstoff, Formgedächtnislegierung, etc..

Die Baueinheit 28 ist über zwei elektrische Leitungswege 29, die längs des Führungsstabes 1 angeordnet sind, mit einem in der Bedieneinheit 3 angeordneten elektrischen Schaltkreis 30 verbunden. Der elektrische Schaltkreis 30 enthält im dargestellten Fall, eine elektrische Energiequelle 31, eine Schaltereinheit 32, die als Aktuator dient und durch den ersten Bedienhebel 19 betätigbar ist, sowie eine elektrische Steuer- und Kontrolleinheit 33.

Anstelle der in Figur 4 illustrierten, als Handgriff ausgebildeten Bedieneinheit 3 ist es ebenso denkbar die Bedieneinheit als Verbindungsflansch zur Adaption an und Betätigung durch eine Robotereinheit auszubilden. In diesem Fall erfolgt die Ansteuerung der im Führungselement 5 integrierten Baueinheit 28 durch ein roboterseitig vorgesehenes Steuersystem, das zugleich die Robotermotorik kontrolliert, um auf diese Weise die Voraussetzungen für ein autonomes Intubieren zu schaffen. Dies setzt selbstverständlich voraus, dass am distalen Bereich des Führungselementes eine geeignete Sensorik angebracht ist. So bietet es sich bspw. an, wie vorstehend bereits erwähnt, durch das Lumen des Führungsstabes oder eines weiteren Lumens durch den Führungsstab ein Beobachtungsmittel zu führen, beispielsweise in Form eines Beleuchtungs-Katheters, eines Kamera-Katheters und/oder eines LIDAR-Sensor unterstützten Katheters.

Auf die Darstellung der zweiten Bedieneinheit 25 sowie des Schub-Zug-Mechanismus 22 an der als Handgriff ausgebildeten Bedieneinheit 3 ist in Figur 4 aus Gründen einer übersichtlichen Darstellung verzichtet worden.

### Bezugszeichenliste

- 1: Führungsstab
- 2: proximales Stabende
- 3: Bedieneinheit, Handgriff
- 4: distales Stabende
- 5: bidirektional verformbares Führungselement
- 6: Längsachse
- 7: proximales Elementende
- 8: distales Elementende
- 9: Unterbrechungs-freie Hülsenwand
- 10: bidirektional verformbarer Abschnitt
- 11: rippenbogenartige Wandabschnitte
- 12: Hülsenwandsteg
- 13: Befestigungsstruktur
- 14,15: Strangabschnitt
- 14p, 15p: Strangabschnittsende
- 16: Aktor
- 17: Drehachse
- 18: Drehelement
- 19: erster Bedienhebel
- 20: Schwenkrichtung
- 21: Konnektor
- 22: Schub-Zug-Mechanismus
- 23: Zahnrad
- 24: Zahnstange
- 25: zweites Bedienelement
- 26: Zahnstange
- 27: Rastmechanismus
- 28: Baueinheit
- 29: elektrische Leitungswege
- 30: elektrischer Schaltkreislauf
- 31: elektrische Energiequelle
- 32: Schaltereinheit
- 33: Steuer- und Kontrolleinheit
- R1, R2: Krümmungsrichtungen

## Patentansprüche

1. Medizinisches Instrument zum Intubieren eines Patienten mit einem einen Hohlkanal einschließenden Endotrachealtubus, kurz ETT, sowie einem längs des Hohlkanals angeordneten, wenigstens in einem axialen Teilbereich plastisch verformbaren Führungsstab mit einem distalen und proximalen Stabende, an dessen distalen Stabende ein bidirektional verformbares Führungselement angebracht ist, mit einem distalen und einem proximalen Elementende, dessen proximales Elementende am distalen Stabende gefügt ist, und das mit einem Steuermittel in Wirkverbindung steht,
**dadurch gekennzeichnet, dass** am proximalen Stabende des Führungsstabes eine Bedieneinheit angebracht ist, mit einem mit dem Steuermittel verbundenen Aktuator sowie mit einem lösbar fest mit dem ETT in Wirkverbindung stehenden Schub-Zug-Mechanismus, durch den der ETT längs des Führungsstabes bewegbar ist.

2. Medizinisches Instrument nach Anspruch 1,
**dadurch gekennzeichnet, dass** das Steuermittel wenigstens zwei Zugkräfte übertragende Strangabschnitte aufweist, mit jeweils einem am distalen Elementende des bidirektional verformbaren Führungselementes fixierten Strangabschnittsende sowie jeweils einem dem am Führungselement fixierten Strangabschnittende gegenüberliegenden Strangendabschnitt, der mit dem Aktuator derart in Wirkverbindung steht oder bringbar ist, dass bei Betätigung des Aktuators beide Strangabschnitte entgegengesetzt zueinander kinematisch auslenkbar sind.

3. Medizinisches Instrument nach Anspruch 2,
**dadurch gekennzeichnet, dass** der Aktuator ein um eine Drehachse drehbar gelagertes Drehelement aufweist, an dem die Strangendabschnitte beider Strangabschnitte in jeweils zur Drehachse gegenüberliegenden Halbebenen mit dem Drehelement in Wirkverbindung stehen.

4. Medizinische Instrument nach Anspruch 2 oder 3,
**dadurch gekennzeichnet, dass** die zwei Zugkräfte übertragenden Strangabschnitte an den Strangendabschnitten einstückig miteinander verbunden sind.

5. Medizinisches Instrument nach einem der Ansprüche 1 bis 4
**dadurch gekennzeichnet, dass** die Bedieneinheit in Art eines Handgriffes oder eines Verbindungsflansches zur Adaption an und Betätigung durch eine Robotereinheit ausgebildet ist.

6. Medizinisches Instrument nach Anspruch 5,
**dadurch gekennzeichnet, dass** der Handgriff einen ergonomisch geformten Umgreifungskörper aufweist,
dass der Aktuator wenigstens ein erstes Bedienelement vorsieht, das am Umgreifungskörper derart ergonomisch angeordnet und ausgebildet ist, dass das erste Bedienelement mittels eines Daumens einer den Umgreifungskörper umschließenden Hand betätigbar ist, und
dass der Schubmechanismus wenigstens ein zweites Bedienelement vorsieht, das am Umgreifungskörper derart ergonomisch angeordnet und ausgebildet ist, dass das zweite Bedienelement mittels eines Zeige- oder. Mittelfingers einer den Umgreifungskörper umschließenden Hand betätigbar ist.

7. Medizinisches Instrument nach Anspruch 6,
**dadurch gekennzeichnet, dass** das zweite Bedienelement in Art eines am Umgreifungskörper angebrachten Hebels ausgebildet ist, der über eine im Umgreifungskörper angeordnete Getriebeeinheit mit einer Schub-Zug-Stange kinematisch verbunden ist, die lösbar fest mit dem proximalen Bereich des ETT gefügt ist, und
dass die Getriebeeinheit bei proximalwärtiger Auslenkung des Hebels in Richtung des Umgreifungskörpers die Schub-Zug-Stange sowie den mit dieser lösbar fest gefügten ETT distalwärts und bei entgegen gerichteter Auslenkung des Hebels die Schub-Zug-Stange sowie den mit dieser lösbar fest gefügten ETT proximalwärts schiebt.

8. Medizinisches Instrument nach Anspruch 7,
**dadurch gekennzeichnet, dass** die Getriebeeinheit ein um eine Drehachse drehbar gelagertes Zahnrad mit Außenverzahnung vorsieht, in die eine mit dem Hebel verbundene Zahnstange sowie die in Art einer Zahnstange ausgebildete Schub-Zug-Stange eingreifen.

9. Medizinisches Instrument nach einem der Ansprüche 2 bis 8,
**dadurch gekennzeichnet, dass** das Führungselement zumindest bereichsweise aus einem biegeelastischen Material gefertigt ist, über eine Längserstreckung vom proximalen zum distalen Elementende mit einer dem Führungselement zugeordneten Längsachse verfügt sowie am distalen Elementende eine Befestigungsstruktur vorsieht, mit einer symmetrisch zur Längsachse orthogonal orientierten Erstreckung, und
dass die distalen Elementenden der zwei Zugkräfte übertragenden Strangabschnitte an jeweils quer zur Längsachse beabstandeten Befestigungsstellen an der Befestigungsstruktur angelenkt sind.

10. Medizinisches Instrument nach Anspruch 1,
**dadurch gekennzeichnet, dass** das bidirektional verformbare Führungselement eine Baueinheit aufweist, die durch elektrische, pneumatische oder hydraulische Steuersignale eine Formänderung des Führungselementes initiiert,
dass das Steuermittel einen mit der Baueinheit verbundenen Schaltkreislauf vorsieht, und
dass der an der Bedieneinheit angebrachte Aktuator Teil des Schaltkreislaufes ist.

11. Medizinisches Instrument nach Anspruch 10,
**dadurch gekennzeichnet, dass** die Baueinheit wenigstens eine der nachfolgenden Komponenten umfasst: mechatronische Baueinheit, elektromotorische Getriebeeinheit, Aktor-Sensor-Einheit, piezoelektrischer Werkstoff, magnetostriktiver Werkstoff, Formgedächtnislegierung, Blähkörper, Fluidleitung.

12. Medizinisches Instrument nach einem der Ansprüche 1 bis 11,
**dadurch gekennzeichnet, dass** der Führungsstab eine Länge besitzt, die wenigstens der Länge des ETTs entspricht.

13. Medizinisches Instrument nach einem der Ansprüche 1 bis 12,
**dadurch gekennzeichnet, dass** der Führungsstab von einem Lumen durchsetzt ist, durch das das Steuermittel verläuft.

14. Medizinisches Instrument nach Anspruch 13,
**dadurch gekennzeichnet, dass** längs des Lumens oder eines weiteren Lumens durch den Führungsstab sowie eines sich längs des Führungselementes erstreckenden Lumens ein Beobachtungsmittel geführt ist.

15. Medizinisches Instrument nach Anspruch 14,
**dadurch gekennzeichnet, dass** das Beobachtungsmittel in Art wenigstens eines der nachfolgenden Komponenten ausgebildet ist: Beleuchtungskatheter, Kamerakatheter, LIDAR-Katheter.
